# EUROPEAN PATENT APPLICATION

(11) **EP 2 804 118 A2**
(43) Date of publication of application: **19.11.2014**
(21) Application number: 14167670.0
(22) Date of filing: 09.05.2014
(51) Int. Cl.: G06F 19/00

(54) **Computer-based System and Method for Presenting and Controlling Access to Medical Information**

(30) Priority: 14.05.2013 US 201313893753
(71) Applicant: Xerox Corporation, Rochester, New York 14644 (US)
(72) Inventor: Xu, Beilei, Penfield, NY New York 14526 (US); Schweid, Stuart A, Pittsford, NY New York 14534 (US); Kehoe, Michael P, Rochester, NY New York 14620-2156 (US); Austin, Paul R, Webster, NY New York 14580 (US)
(74) Representative: Skone James, Robert Edmund

(57) **Abstract**

A computer-based method for presenting medical information, including: storing, in at least one memory element (104) of at least one computer, computer readable instructions; and executing, using at least one processor for the at least one computer, the computer readable instructions to: receive an input selecting a medical condition; identify a portion of an electronic medical record (EMR) (116) for the patient, specific to the patient, related to the medical condition; generate a video including at least one video segment with the portion of the EMR; designate at least one first level of access for the at least one video segment; and control access to the at least one video segment according to the at least one first level of access.

## Description

The present disclosure relates to a system and method for automatically generating a video including at least one video segment related to personal information from an electronic medical record for a patient. The system and method also designate respective levels of access for the video segment and possible users of the system and method and control access to the video according to the respective levels of access.

It is expensive to provide standard healthcare information via personal interaction with a healthcare professional. Many videos exist that describe various medical conditions and treatments; however, such videos do not incorporate patient-specific information and thus provide minimal help to the patient with respect to understanding their particular situation and complying with a particular regimen or therapy. Further, it is difficult for a patient with a non-medical background to assess the quality and relevance of videos on the internet.

U.S. Patent Application Publication 2008/0310816 personalizes a medical video, for example, a sonogram, by adding decorative, non-medically related information. This publication also teaches remote sharing of video among medical practitioners. U.S. Patent Application Publication US2012/0253848 teaches access to medical information in the system in a real time communication between a medical practitioner and patient. This application is closely related to telemedicine. Neither of these references addresses the problem of providing patient-specific information in a video.

According to aspects illustrated herein, there is provided a computer-based method for presenting medical information, including: storing, in at least one memory element of at least one computer, computer readable instructions; and executing, using at least one processor for the at least one computer, the computer readable instructions to: receive an input selecting a medical condition; identify a portion of an electronic medical record (EMR) for the patient, specific to the patient, related to the medical condition; generate a video including at least one video segment with the portion of the EMR; designate at least one first level of access for the at least one video segment; and control access to the at least one video segment according to the at least one first level of access.

According to aspects illustrated herein, there is provided a computer-based apparatus for presenting medical information, including: at least one computer including at least one memory element configured to store computer readable instructions; and at least one processor configured to execute the computer readable instructions to: receive an input selecting a medical condition; identify a portion of an electronic medical record (EMR) for the patient, specific to the patient, related to the medical condition; generate a video including at least one video segment with the portion of the EMR; designate at least one first level of access for the at least one video segment; and control access to the at least one video segment according to the at least one first level of access.

According to aspects illustrated herein, there is provided a computer-based method for presenting medical information, including: storing, in at least one first memory element of at least one first computer for a first entity: computer readable instructions and a first level of access for a person other than a patient; storing, in at least one second memory element of at least one second computer for a second entity providing healthcare a health product or service to the patient, an electronic medical record (EMR) for the patient; and executing, using at least one first processor for the at least one first computer, the computer readable instructions to: access the EMR in the at least one second memory element; generate a video including a video segment including information, specific to a patient, from the EMR; assign a second level of access to the video; enable access to the video segment for the patient; and enable access video to the video segment for the person when the second level of access is the same as the first level of access.

According to aspects illustrated herein, there is provided a computer-based apparatus for presenting medical information, including: at least one first computer, for a first entity, including at least one first processor and at least one first memory element configured to store computer readable instructions and a first level of access for a person other than a patient; and at least one second computer for a second entity providing healthcare a health product or service to the patient including at least one second memory element configured to store an electronic medical record (EMR) for the patient. The at least one first processor is configured to execute the computer readable instructions to: access the EMR in the at least one second memory element; generate a video including a video segment including information, specific to a patient, from the EMR; assign a second level of access to the video; enable access to the video segment for the patient; and enable access to the video segment for the person when the second level of access is the same as the first level of access.

Various embodiments are disclosed, by way of example only, with reference to the accompanying schematic drawings in which corresponding reference symbols indicate corresponding parts, in which:
Figure 1 is a schematic block diagram of a computer-based apparatus for presenting medical information; and,
Figure 2 is a pictorial representation of a user interface for the apparatus of Figure 1;
Figure 3 is a pictorial representation of user interface for the apparatus of Figure 1; and,
Figure 4 is a schematic representation of example configurations of a computer-based apparatus for presenting medical information.

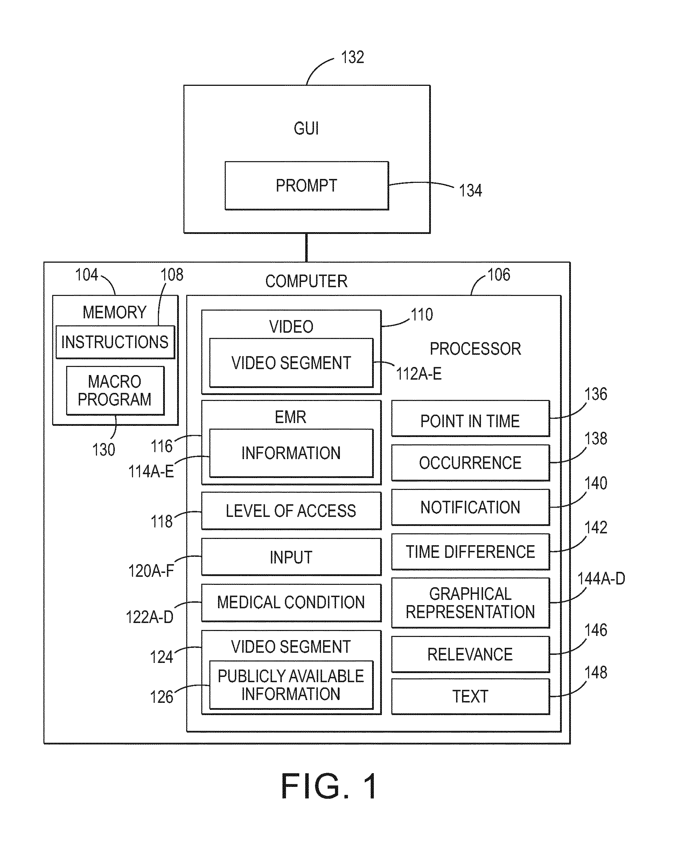

Figure 1 is a schematic block diagram of computer-based apparatus 100 for presenting medical information. Apparatus 100 includes at least one computer 102 with at least one memory element 104, and at least one processor 106. The memory element is configured to store computer readable instructions 108. Processor 106 is configured to execute the computer readable instructions to generate video 110 including at least one video segment 112 including information 114, specific to a patient, from electronic medical record (EMR) 116 for the patient. In an example embodiment, processor 106 is configured to execute the computer readable instructions to generate at least one audio segment 117 for at least one video segment 112 By "specific to the patient" we mean the information is regarding specifics of a patient condition and that the information is not generic to a condition which the patient may have. For example, if a patient has diabetes, information 114 could include specific laboratory results for the patient, while information generic to the patient could discuss possible significance of laboratory results in general. By "EMR" we mean any electronic data, generated by a healthcare provider, related to healthcare for the patient. By "healthcare provider" we mean: a medical practitioner, such as a medical doctor, nurse practitioner, physician's assistant, nurse, or physical therapist; a pharmacist or entity providing medication; or a mental health practitioner, such as a counselor, therapist, or psychologist. Processor 106 is configured to execute the computer readable instructions to designate at least one level of access 118 for segment 112 and control access to segment 112 according to level 118. In the discussion that follows, it should be understood that stating that the processor implements an action or that the processor is configured to implement an action is analogous to stating that the processor 106 is configured to execute the computer readable instructions to implement or execute the action.

Processor 106 is configured to execute the computer readable instructions to: receive input 120A selecting medical condition 122A, select stock video segment 124 including publicly available information 126 regarding the medical condition (or generates segment 124 from publicly available material); identify information 114A from the EMR, specific to the patient, related to medical condition 122A; and generate video segment 112A including portion 114A; and include segment 112A in video 110. In an example embodiment, processor 106 generates audio segment 117A for segment 112A. By "publicly available information" we mean information that is not specific to the patient and provides general/generic information regarding a medical condition, for example, open source information or information for an advocacy group such as the American Cancer Society. By "generate a segment" or "generate a video segment" we mean import material or information, for example from the EMR, put the material or information into a suitable format, and create a video segment incorporating the formatted material or information. The material or information can include, but is not limited to, any information typically included in an EMR, such as medical images, videos, laboratory results, and notes or dictation from a healthcare provider.

Further detail regarding the creation of video segments 112 and 124 is provided in commonly owned U.S. Patent Application Nos. 13/150,450, 13/150,462, and 13/484,627, which applications are incorporated in their entirety herein.

For example, a healthcare provider wishes to provide the patient with video 110 regarding a particular medical condition, such as type II diabetes, suffered by the patient. The provider selects type II diabetes (provides an input to apparatus 100 selecting type II diabetes), the processor obtains generic information 126 regarding type II diabetes, searches the patient's EMR for information 114B, such as lab results, specific to the patient and type II diabetes, generates video segments 124 and 112A, and add segments 124 and 112A to video 110. In an example embodiment, apparatus 100 is configured to receive, from the health provider, input 120B regarding segment 112 and add input 120B to video 110. For example, input 120B can be an audio segment (voice over), an analysis of a portion of the EMR, an evaluation of a status of the patient, feedback for the patient, or a diagnosis.

In an example embodiment, video 110 includes a plurality of video segments 112 and the at least one processor is configured to: assign level of access 118A to a person other than the patient; assign a respective level of access 118 to each video segment 112 in the plurality of video segments 112; and enable, for the person, access to a video segment 112 only when the video segment is assigned level of access 118A. In an example embodiment, processor 106 is configured to assign a respective level of access 118 to each person in a plurality of persons other than the patient and enable, for each person, access to a video segment 112 only when the video segment is assigned the same level of access 118 as is assigned to the person. That is, as further described below, apparatus 100 enables persons other than the patient to access video 110, but strictly controls that access.

In an example embodiment, processor 106 is configured to facilitate creation of video 110, for example by use of macro program 130. For example, in response to input 120, program 130 displays, for example, on graphical user interface 132, prompt 134 to provide information, from the EMR related to medical condition 122. The healthcare provider identifies an applicable portion of the EMR, and provides input 120C, accepted by processor 106, identifying the portion of the EMR related to the medical condition. The processor then includes information from that portion of the EMR in segment 112. As necessary, the processor generates a video segment from that portion of the EMR.

Apparatus 100 also includes notification functionality. In an example embodiment, the processor is configured to: identify information 114C in the EMR added since a specified point in time 136; determine last occurrence 138 of an access to the video by at least one user; and send notification 140 to the at least one user according to time difference 142 between occurrence 138 and point in time 136. In an example embodiment, the at least one user includes the patient and one or more optional additional persons other than the patient. The processor is configured to: assign levels of access 118B/C/D to the patient and the additional persons, respectively; generate video segment 112B including the information added to the EMR; receive input 120D designating level of access 118E for the video segment; and send notification 142 to the patient and the additional persons when level 118E is the same as level 118 for the patient or the additional person(s). For example, if the additional person is the healthcare proxy for the patient and level 118E is a level of access restricted to the patient and the patient's healthcare proxy, then notification 142 is sent to the patient and the healthcare proxy only. In an example embodiment, processor 106 generates audio segment 117B for segment 112B.

In an example embodiment, apparatus 100 automatically updates video 100. For example, processor is configured to: identify information 114D being added to the EMR; generate video segment 112C including graphical representation 144A of information 114D; and add video segment 112D to segment 112. In an example embodiment, processor 106 generates audio segment 117C for segment 112C. In an example embodiment, processor 106 generates audio segment 117D for segment 112D. A graphical representation is described further below. In an example embodiment, the processor is configured to execute the computer readable instructions to: identify relevance 146 of information 114D to a medical condition 122B included/described in the EMR; identify information 114E in the EMR related to medical condition 122B; generate video segment 112E including graphical representation 144B of information 114E; and add video segment 112E to video 110. That is, apparatus 100 parses information added to the EMR, identifies existing information with relevance to the newly added information, and modifies segment 112 to incorporate the newly added information and relevant existing information. For example, information 114E may not have been relevant to condition 122B prior to the addition of information 114D. In an example embodiment, processor 106 generates audio segment 117E for segment 112E. By "graphical representation" we mean a representation that relies on visual elements, such as pictures, videos, diagrams, charts, or graphs, rather than text. It should be noted that text can be included, for example, to label portions of a chart.

In an example embodiment, apparatus 100 automatically generates visual content for video 110. For example, to generate video 110, the processor is configured to: receive input 120E identifying medical condition 122C; identify, in the EMR, graphical representation 144C related to medical condition 122C (for example, a graph showing lab results); generate video segment 112F including graphical representation 144C; and include video segment 112F in video 110. Thus, apparatus 100 automatically incorporates graphical representations, which are generally more helpful than text in conveying information to a patient.

In an example embodiment, to generate video 110, the processor is configured to: receive a input 120F identifying medical condition 122D; identify, in the EMR, text 148 related to medical condition 122D; generating video segment 112G including graphical representation 144D of text 150; and include video segment 112G in video 110. Thus, apparatus 100 converts text to more readily understood graphical representations. For example, if text 150 includes values for a medical parameter with respect to time, for example pulse rate during a stress test, or values for a one medical parameter with respect to another medical parameter, for example, ratios of elements in blood chemistry samples, the apparatus converts the text to a chart or graph, for example, that visually displays the information in question. In another example, if a patient's EMR record reflects periodic measurements of blood glucose or body weight, a longitudinal graph of such measurements may be illuminating.

The following provides further detail regarding apparatus 100 and a method employing apparatus 100. Apparatus 100 enables a user, for example a healthcare provider, to select the video content to be shared with the patient, and the patient's designated persons, and to receive prompt notification when contents have been created or updated. Example use of apparatus 100 includes the following steps:
1) generating/updating all video segments including stock video, such as video 124, and personalized segments, such as segments 112, based on a patient's most recent EMR;
2) tagging all stock segments as "customized" and all created personalized segments as "personalized." This tagging can be achieved by creating a metadata field in each video segment;
3) Checking/verifying date/time of video segments that have been accessed by the user;
4) Sending, for example via email or a hardcopy with QR code, a link of a user interface to the user where the user can "customize" or "personalize" a video playlist by choosing components tagged with "customized" or "personalized." For example, "customize" only includes components tagged with "customized" while "personalize" creates a video playlist including any components tagging as either "personalized" or "customized".

A personalized medical video, such as video 110, can consist of stock information, such as information 126, in various media types, such as segment 124, as well as personalized information, such as segments 112, created based on a patient's EMR or entered by a healthcare professional. The purpose of the video is to assist patients and their caregivers to understand medical conditions and possible treatments. For example, for a diabetic patient, there are many stock videos available on the web that explain the basic causes and symptoms such as what type I or II is and what are the physiological processes that affect the glucose level in the blood stream. This information already exists in the public domain or a video database in a healthcare organization. However, the personalized video creation tool filters the database and selects only those segment(s) pertinent to a specific patient. For example, for a patient with type II diabetes, only information, e.g. the symptoms, tests or treatments, related to type II is included. Those segments will be tagged as "customized" as they have been selected (i.e., customized) for the specific patient from a vast pool of related information in public. As another example, warnings or precautions for a female regarding a prescription medication are not included in a customized segment for a male patient.

On the other hand, for patients to better understand their specific conditions and treatments, their personal medical information, for example, lab results/medical images/medications, also can be included in the video. Those video segments can be synthesized from text files, lab result tables, still medical images, medical videos, and doctor dictated audio files. Such segments are tagged as "personalized".

When a patient decides to share his/her personalized video, he/she might want to share those personalized video components with some individuals, and only the customized segments with others. For example, currently, MPEG4 supports a variety of metadata and methods for encoding information in a video. For example, there are tags that store the creation time and modification time of a video that are present in virtually every MP4 video. Additionally, there are metadata tags that can store the name, description and a commentary of a video. If even greater extensibility is needed, the MPEG container has user defined tags (uuid) that can be generated to store metadata. Finally, there is a provision, via the use of the "free" tag, to store metadata using any user defined structure or paradigm. For example, to enable user's choice of segments to share, each video segment in a personalized medical video can be tagged as "customized" or "personalized." The segments that are pulled from stock database will be tagged as "customized" as they have been selected, that is customized, for the specific patient from a vast pool of related information while the segments that are created using patient's personal medical information will be tagged as "personalized".

It is possible to ascertain both the last modification time and the last viewing time of a video segment by querying the file system, for example, NTFS or ZFS. Additionally, since a user might view only certain video segments during any personalized video viewing, for example only the customized parts, it is possible to make the determination on a video segment-by-segment basis and not just on a full video only. Therefore, if desired, only the video segments altered since the last patient access to the video need to be viewed by the user. This process not only potentially reduces the number of notices sent to the patient for newly created content, it also reduces the amount of effort in the healthcare provider's office to update the video content. The update can be initiated by a healthcare provider manually based on need or driven by an event such as an update in a patient's EMR, for example a new lab result, or a follow-up to a recent visit to the healthcare provider's office.

The steps described above focus on actions in a healthcare provider's office where the video components are created or updated. In addition, all video components can be written onto a DVD to be shared with the patient and his/her caregivers. If a user prefers to have control over the content being shared, a user interface, described below, can be presented to the user.

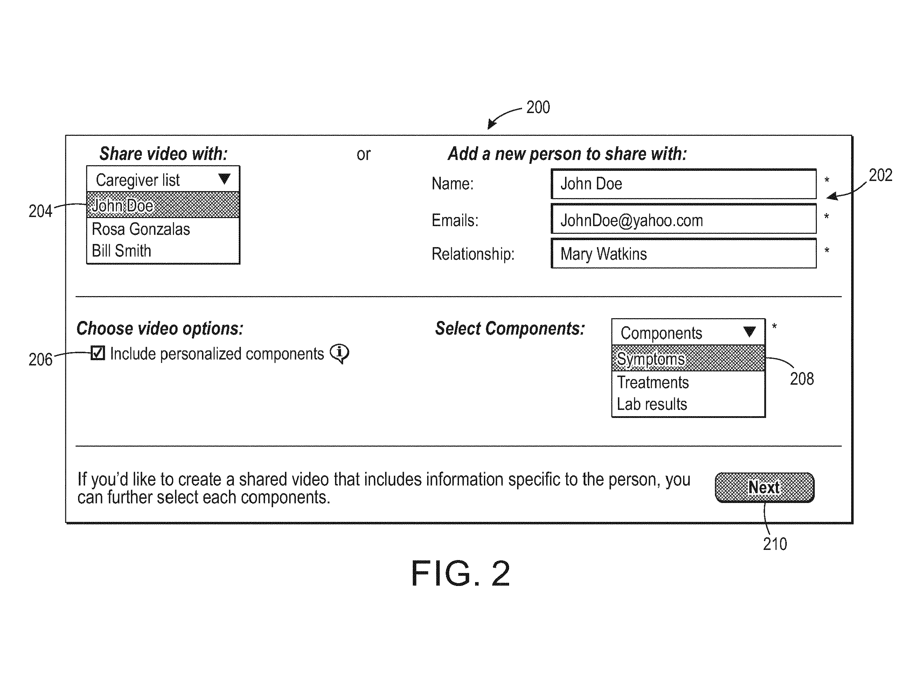

Figure 2 is a pictorial representation of user interface (UI) 200 for apparatus 100 of Figure 1. The following should be viewed in light of Figures 1 and 2. With UI 200, the user can choose the individual component, or video segment, to be shared, for example, based on whether the component or video segment is "personalized" content, or only "customized." In an example embodiment, UI 200 includes fields 202 in which a patient/user can enter information, such as an address (email or postal) identifying a person(s) s/he would like to share some or all of video 110 with. Fields 204 lists persons selected to receive some or all of video 110. Check box 206 is used to designate a selected name in fields 204 as receiving segments 112. Fields 208 are used to select respective video segments related to specific areas of concern. To determine how to share various video segments of video 110 with the person selected in field 204, button 210 is selected. After choosing what video content will be shared, apparatus 100 automatically sends the designated portions of video 110 as described above.

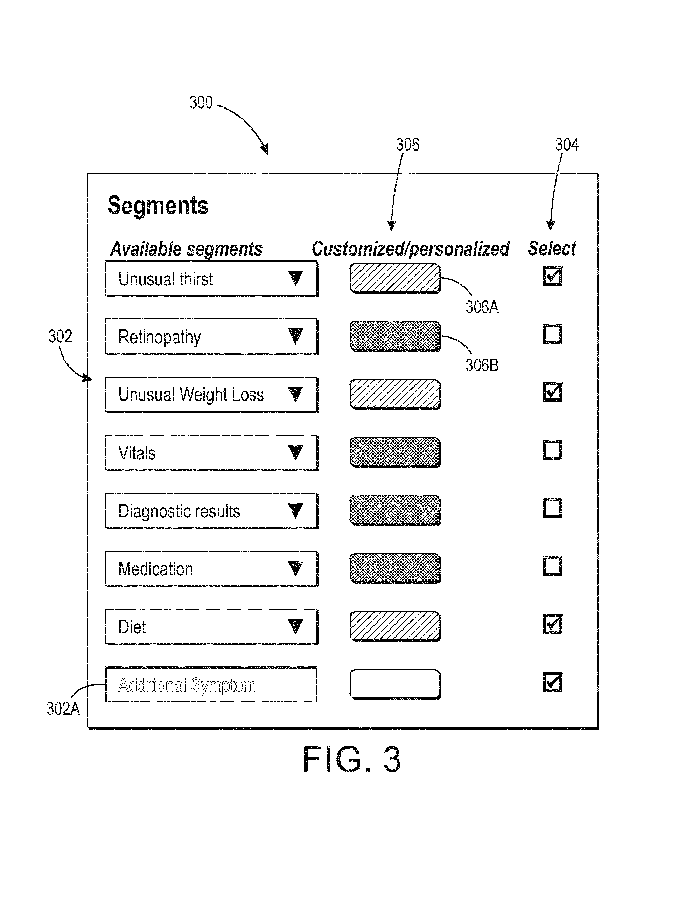

Figure 3 is a pictorial representation of user interface (UI) 300 for apparatus 100 of Figure 1. The following should be viewed in light of Figures 1 through 3. For more fine-grained control, shared content can be selected on a video segment-by- video segment basis, for example, as shown in Fig. 3. As noted above, UI 300 is opened for the person selected in field 204 of Figure 2. Fields 302 shows categories of available video segments and are populated, for example, based on selected fields 208 in Figure 2. The level of access is selected using checkboxes 304 and the selected access is shown in fields 306. If the checkbox for a segment is unchecked, then only customized boxes are made available. If the checkbox for a segment is checked, then both customized and personalized video segments are made available. For example in Figure 3, field 306A shows that both customized and personalized video segments for unusual thirst are shared with John Doe, while field 306B shows that only customized video segments for retinopathy are shared with John Doe. Empty field 302A enables the patient to also include a personalized segment for the particular person.

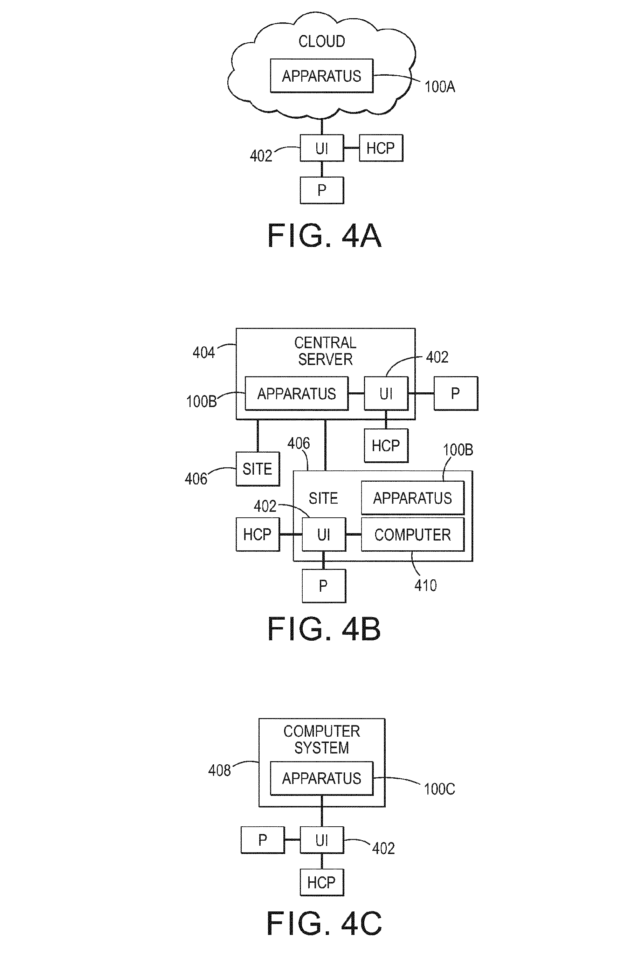

Figures 4A through 4C are schematic representation of example configurations of a computer-based apparatus for presenting medical information, such as apparatus 100. In the example embodiment of Figure 4A, apparatus 100A is cloud-based and patient P and healthcare provider HCP interface with the apparatus user interface 402, which can be web based. Any known cloud functionality or user interface known in the art can be used. For example, the patient can bring up UIs 200 and 300 via interface 402.

In the example embodiment of Figure 4B, apparatus 100B is part of central server 404 serving a network of sites 406. The patient and HCP can interface with apparatus 100B via interface 402 which can be associated with the central server or one of sites 406.

In the example embodiment of Figure 4C, apparatus 100C is part of computer system 408 for a HCP. The patient can interface with apparatus 100C via interface 402.

In an example embodiment, the HCP is providing diagnostic and treatment services for the patient, for example, the HCP is a medical doctor or nurse practitioner. In such a case, video 110 can be provided as a follow-up to a visit with the HCP and/or can be updated based on information such as lab results received by the HCP. For example, if the medical condition for the patient is type II diabetes, segment 124 can be generic information and segment 112 can be lab results showing blood sugar levels and specific instruction from the doctor regarding diagnosis, prognosis, exercise and diet.

In an example embodiment, the HCP is providing medication or healthcare-related equipment to the patient; the HCP is a pharmacist, for example. In such a case, video 110 can be provided with a prescription being filled or can be follow-up information for a prescription already filled. For example, segment 124 can be generic information about side effects and segment 112 can specific instructions regarding dosage and timing for the medication.

In an example embodiment, apparatus 100 is used by a third party, such as an insurance company, involved with overseeing or funding healthcare services. For example, the third party could be managing apparatus 100A or 100B in Figures 4A and 4B, respectively. For example, in Figure 4B, the functionality of apparatus 100B can be wholly or partly in server 404 for the insurance company and a portion of the functionality of apparatus 100B is at site 406 for a HCP. Server 404 communicates as needed with computer 410, for example, to obtain information from an EMR stored in computer 410. The patient can use interface 402 associated with server 404 or site 406.

Thus, apparatus 100 and a method using apparatus 100 provide a cost effective way to provide general and specific healthcare information, selected and recommended by a medical provider, in the form of personalized video 110 that a patient can view multiple times and share with concerned parties. For example, apparatus receives an input selecting a medical condition and without further prompting or intervention by the medical provider parses the EMR of the patient to identify portions of the EMR relevant to the condition. Then, again without prompting or intervention by the medical practitioner, automatically generates a video segment including the patient-specific information. To ensure the privacy of the patient and to meet the wishes and requirements of the patient regarding access to the video segment, apparatus automatically governs control to the video segment according to levels of access established by the patient. Thus, with only the input of a medical condition and levels of desired access, apparatus 100 automatically creates a video segment that can provide critically useful, or even necessary, information for the patient and persons associated with the patient. For example, the video segment can provide information to help the patient and selected persons understand the diagnosis, prognosis, and treatment of the condition in an easily accessed and understood format, and in a format that can be viewed whenever necessary or desired. Thus, compliance of the patient with treatment is optimized. Further, understanding of the condition by those associated with the patient is optimized, which can ensure that support or intervention can be provided as needed.

Medical video 100 combines stock tutorial video with information tailored for a specific patient. Healthcare providers can use the service to convey general as well as patient-specific information about conditions, symptoms, treatment, and prognostics. Patients benefit from the service by being informed about diagnosis, treatment and post-treatment care as applicable to their individual condition. Patients further benefit in that the information service is repeatable (can be viewed multiple times), and can be shared with others, for example, trusted friends or family members, who are assisting the patient with healthcare decisions.

Thus, apparatus 100 provides a synergistic approach that is not possible using known systems or approaches. For example, as noted above, U.S. Patent Application Publication 2008/0310816 personalizes a medical video, for example, a sonogram, by adding decorative, non-medically related information. However, the system of this application has no means of or teaching related to automatically identifying information regarding a medical condition from an EMR and automatically generating a video including that information. Further, this application has no teachings what so ever regarding controlled access to a video as selected by the patient. This publication also teaches remote sharing of video among medical practitioners. Again, this has no relevance to a personalized video for use by a patient.

U.S. Patent Application Publication US2012/0253848 teaches access to medical information in the system in a real time communication between a medical practitioner and patient. This application is closely related to telemedicine. However, the system of this application has no means of or teaching related to automatically identifying information regarding a medical condition from an EMR and automatically generating a video including that information. Further, this application has no teachings what so ever regarding controlled access to a video as selected by the patient.

## Claims

1. A computer-based method for presenting medical information, comprising:
storing, in at least one memory element of at least one computer, computer readable instructions; and,
executing, using at least one processor for the at least one computer, the computer readable instructions to:
receive a first input selecting a medical condition;
identify a portion of an electronic medical record (EMR) for the patient, specific to the patient, related to the medical condition;
select a first video segment including publicly available information regarding the medical condition;
generate a video including at least one video segment with the portion of the EMR and the first video segment;
designate at least one first level of access for the at least one video segment;
designate at least one second level of access for the first video segment;
control access to the at least one video segment according to the at least one first level of access; and
control access to the fist video segment according to the at least one second level of access.

2. The computer-based method of claim 1, wherein:
the at least one video segment includes a plurality of first video segments, the method further comprising executing, using the at least one processor, the computer readable instructions to:
assign a first level of access to a person other than the patient;
assign a respective level of access to each first video segment in the plurality of first video segments; and,
enable, for the person, access to said each first video segment only when said each first video segment is assigned the first level of access.

3. The computer-based method of claim 1 or claim 2, further comprising executing, using the at least one processor, the computer readable instructions to:
assign a respective level of access to each person in a plurality of persons other than the patient; and,
enable, for said each person, access to said each first video segment only when said each first video segment is assigned the respective level of access.

4. The computer-based method of any of the preceding claims, further comprising executing, using the at least one processor, the computer readable instructions to:
receive, from a healthcare provider, a second input selecting a medical condition applicable to the patient;
display a prompt to provide information, from the EMR, related to the medical condition;
accept a third input identifying a portion of the EMR related to the medical condition; and,
include the portion of the EMR in the at least one video segment.

5. The computer-based method of any of the preceding claims, further comprising:
identifying information in the EMR added since a specified point in time;
determining a last occurrence of an access to the video by at least one user; and,
sending a notification to the at least one user according to a first time difference between the last occurrence and the specified point in time.

6. The computer-based method of claim 5, wherein:
the at least one user includes the patient and first and second persons other than the patient, the method further comprising executing, using the at least one processor, the computer readable instructions to:
assign first, second, and third levels of access to the patient and the first and second persons, respectively;
generate a first video segment including the information added to the EMR;
receive a second input designating a fourth level of access for the first video segment; and,
send the notification the patient, the first, or the second person when the fourth level is the same as the first, second, or third level, respectively.

7. The computer-based method of any of the preceding claims, further comprising executing, using the at least one processor, the computer readable instructions to:
identify first information being added to the EMR;
generate a first video segment including a first graphical representation of the first information; and,
add the first video segment to the at least one video segment.

8. The computer-based method of claim 7, further comprising executing, using the at least one processor, the computer readable instructions to:
identify relevance of the first information to a first medical condition included in the EMR;
identify second information in the EMR related to the first medical condition;
generate a second video segment including a second graphical representation of the second information; and,
add the second video segment to the at least one video segment.

9. The computer-based method of any of the preceding claims, wherein generating the video includes one or more of:
a) receiving a second input identifying a medical condition;
identifying, in the EMR, a graphical representation related to the medical condition;
generating a first video segment including the graphical representation; and,
including the first video segment in the at least one video segment, and
b) receiving a second input identifying a medical condition;
identifying, in the EMR, text related to the medical condition;
generating a first video segment including a graphical representation of the text; and,
including the first video segment in the at least one video segment, wherein generating the video preferably further includes:
generating at least one audio segment for the at least one video segment;
generating a first audio segment for the first video segment; and,
including the first audio segment in the at least one audio segment.

10. A computer-based apparatus for presenting medical information, comprising:
at least one computer including:
at least one memory element configured to store computer readable instructions; and,
at least one processor configured to execute the computer readable instructions to:
receive a first input selecting a medical condition;
identify a portion of an electronic medical record (EMR) for the patient, specific to the patient, related to the medical condition;
generate a video including at least one video segment with the portion of the EMR;
designate at least one first level of access for the at least one video segment;
control access to the at least one video segment according to the at least one first level of access;
identify information in the EMR added since a specified point in time;
determine a last occurrence of an access to the video by at least one user; and,
send a notification to the at least one user according to a first time difference between the last occurrence and the specified point in time.

11. The computer-based apparatus of claim 10, wherein the at least one processor is configured to execute the computer readable instructions to:
identify first information being added to the EMR;
generate a first video segment including a first graphical representation of the first information; and,
add the first video segment to the at least one video segment.

12. The computer-based apparatus of claim 10 or claim 11, wherein generating the video includes:
receiving a second input identifying a medical condition;
identifying, in the EMR, a graphical representation related to the medical condition;
generating a first video segment including the graphical representation; and,
including the first video segment in the at least one video segment.

13. The computer-based apparatus of any of claims 10 to 12, wherein generating the video includes:
receiving a second input identifying a medical condition;
identifying, in the EMR, text related to the medical condition;
generating a first video segment including a graphical representation of the text; and,
including the first video segment in the at least one video segment.

14. A computer-based method for presenting medical information, comprising:
storing, in at least one first memory element of at least one first computer for a first entity:
computer readable instructions; and,
a first level of access for a person other than a patient;
storing, in at least one second memory element of at least one second computer for a second entity providing healthcare a health product or service to the patient, an electronic medical record (EMR) for the patient; and,
executing, using at least one first processor for the at least one first computer, the computer readable instructions to:
access the EMR in the at least one second memory element;
generate a video including a video segment including information, specific to a patient, from the EMR;
assign a second level of access to the video;
enable access to the video segment for the patient; and,
enable access video to the video segment for the person when the second level of access is the same as the first level of access.

15. A computer-based apparatus for presenting medical information, comprising:
at least one first computer, for a first entity, including:
at least one first processor; and,
at least one first memory element configured to store:
computer readable instructions; and,
a first level of access for a person other than a patient; and,
at least one second computer for a second entity providing healthcare a health product or service to the patient including at least one second memory element configured to store an electronic medical record (EMR) for the patient, wherein:
the at least one first processor is configured to execute the computer readable instructions to:
access the EMR in the at least one second memory element;
generate a video including a video segment including information, specific to a patient, from the EMR;
assign a second level of access to the video;
enable access to the video segment for the patient; and,
enable access to the video segment for the person when the second level of access is the same as the first level of access.
